# EUROPEAN PATENT APPLICATION

(11) **EP 4 282 446 A1**
(43) Date of publication of application: **29.11.2023**
(21) Application number: 22175829.5
(22) Date of filing: 27.05.2022
(51) Int. Cl.: A61L 27/26, A61L 27/38, A61L 27/44, A61L 27/54, A61L 27/56

(54) **BIOACTIVE THREE-DIMENSIONAL SCAFFOLD FOR CARTILAGE REPAIR**

(71) Applicant: Lietuvos sveikatos mokslu universitetas, 44307 Kaunas (LT)
(72) Inventor: Ma iulaitis, Justinas, LT-44307 Kaunas (LT); Ma iulaitis, Romaldas, LT-44307 Kaunas (LT); Gudas, Rimtautas, Kaunas (LT)
(74) Representative: Pakeniene, Ausra

(57) **Abstract**

Invention is a bioactive three-dimensional scaffold, comprising electrospinned polycaprolactone material supplemented with polylactic-glicolic acid. The scaffold is augmented with magnesium hydroxide particles, for antiacid properties, which counteract acidifications caused by polymer hydrolysis. The augmented scaffold is characterized by antiacid properties for cartilage tissue engineering during the scaffold degradation.

## Description

### TECHNICAL FIELD

The invention relates to a bioactive three-dimensional scaffold for use for cartilage regeneration.

### BACKGROUND ART

Human articular cartilage is a tissue that lacks blood circulation and innervation, so it has very limited or no natural self-healing potential when it is damaged.

Specialized orthopedic surgery questionnaires have shown that patients with joint cartilage lesions have significantly worse pain and social function compared to the general population. Until now, there is no consensus on exactly how to treat the pathology of joint cartilage lesions, and new drugs and treatments are constantly being sought. The result of currently used surgical procedures in the world is usually poor-quality fibrin cartilage, which has inferior mechanical and physiological properties compared to natural hyaline cartilage. As a result, the fibrin cartilage tends to break down again after a while, and the patient experiences all their previous symptoms.

Knee pains bother about 25 percent of all adult patients (18-65 years). In case of knee pain, arthroscopic surgery is performed to clarify the diagnosis. Clinical trials have found that in 63% of patients who have undergone diagnostic arthroscopic surgery, joint cartilage lesions are found. Of these, 20 % are large (> 5cm²) joint cartilage lesions.

Thus, although all patients with joint cartilage lesions are eligible for regenerative therapy, they are essential for one in five patients diagnosed with joint cartilage lesions arthroscopically. It is also known that about 40 % patients who do not complain of typical symptoms of joint cartilage lesions, are found to have cartilage lesions in the knee during magnetic resonance imaging.

Microfracture/ micro drilling has been a first-choice treatment for less physically active patients, who are diagnosed with small (2 cm²) focal chondral or osteochondral defects [3,4] However, clinical improvement gradually deteriorates after 2 years, usually because of partial fibrocartilaginous repair tissue, incomplete fill up of the defect and intralesional osteophytes. Additionally, poorer clinical outcome was reported after microfracture of multiple AC defects, when compared to patients with a single defect per knee.

Different treatment methods to manage a sustained osteochondral injury have been introduced [6], however complete restoration of cartilage tissue has not yet been achieved. Microfracture employs the underlying subchondral layer of the bone marrow. Penetration of the subchondral layer, results in the outflow of the bone marrow and cartilage restorative cells, that aid in cartilage repair. Recently, a combinatory treatment of microfracture and an outflow from the bone marrow covering scaffold have progressed rapidly and currently hold a promise as a useful tool in the field of regenerative orthopedics and especially, cartilage repair.

Scaffold biomechanical and physical features in vitro are vital for good clinical outcome [1, 2]. Customization of scaffolds with highly predictable physical characteristics, such as mechanical rigidity, pore size, porosity and pore interconnectivity is enabled by the ability to adjust parameters of synthetic polymeric feed material [3, 4, 5].

International patent application No. PCT/US2018/064570 (publication No WO2019113511A1) discloses electrospun fibers-based scaffolds for repair and regrowth of hyaline cartilage i.e., for treating articular or hyaline cartilage damage or injury using biocompatible electrospun polymer fibers-based scaffold. Main disadvantages of the disclosed scaffold are poor biocompatibility, loss of mechanical and tensile strength after application, lack of cartilage regeneration supporting properties.

Polycaprolactone - IUPAC name poly (hexane-6-lactone) (PCL) is a biodegradable polyester with a low melting point (about 60°C) [6]. This polymer is resistant to mechanical impact. Polycaprolactone is biodegradable and biocompatible. In humans, caprolactone is biodegraded by hydrolysis of ester linkages [7, 8]. D-L poly (glycolic acid), also known as PLG or PLGA (Poly (D, L-lactide-co-glycolide)), is a biodegradable, human-compatible copolymer [8, 9].

However, the synthetic biodegradable polymers have been reported to cause the acidification of the cartilage tissue. They also induced loss of chondrogenic phenotype and the inflammatory responses in vitro [8].

PLGA degradation/ hydrolysis in vivo, causes acidification in the vicinity of the scaffold, thus resulting in the cell toxicity and local inflammation [10]. After the degradation, lactic and glycolic acid are formed as by-products, which in turn negatively affect cell growth, proliferation and tissue regeneration.

Magnesium hydroxide ((Mg(OH)₂) is an inorganic compound that is used as a major component of antacids to treat several symptoms such as indigestion or heartburn. It has very low solubility in water, but it can easily dissolve in the presence of acid. Such properties may allow Mg(OH)₂ to neutralize the acidic degradation product of polymer composites. After neutralization by Mg(OH)₂, abundant magnesium ions are less cytotoxic because of their predominant existence in the body (bone, muscle, and soft tissue). Under in vitro conditions, a high concentration of extracellular calcium ion provokes the plasma membrane damage, thereby leading to cytotoxicity, whereas magnesium ion is reported to be relatively nontoxic [20]. Furthermore, magnesium ions could inhibit the vascular calcification [11, 12].

Structural parameters, such as mechanical rigidity, pore size, porosity and pore interconnectivity, provide a homogenous distribution of inflow of host bone marrow cells and nutrient transfer within the bioactive three-dimensional scaffold [13, 14]. In addition, phenotype formation and superior production of ECM proteins can be influenced by the custom design of pore morphology. Scaffold biomechanics can be similarly affected by the surrounding liquid medium, especially the soft natural collagen-based scaffolds, thus reducing biomechanical properties of the scaffold prior to implantation. The ability of bone marrow stem cells to effectively adhere to the scaffold is highly dependent on cell viability. In addition, viable cell proliferation is indicative of the ability to deposit ECM on the scaffold, thus identification of optimal biomaterial morphological parameters is essential for specific cellular processes. Similarly, an acidic scaffold potency in vitro is established by superior regulation of the surrounding pH regulation, while the scaffold is degrading.

The present invention is dedicated to overcoming of the above shortcomings and for producing further advantages over prior art.

### SUMMARY OF INVENTION

Invention is a bioactive three-dimensional scaffold, comprising electrospinned polycaprolactone material supplemented with polylactic-glicolic acid. The scaffold is augmented with magnesium hydroxide particles, for antiacid properties, which counteract acidifications caused by polymer hydrolysis.

The augmented scaffold is characterized by antiacid properties for cartilage tissue engineering during the scaffold degradation.

**Technical problem.** PLGA degradation/ hydrolysis in vivo, causes acidification in the vicinity of the scaffold, thus resulting in the cell toxicity and local inflammation. After the degradation, lactic and glycolic acid are formed as by-products, which in turn negatively affect cell growth, proliferation and tissue regeneration.

**Solution to problem.** A three-dimensional scaffold, comprised of the electrospinned polycaprolactone material supplemented with polylactic-glicolic acid. A scaffold is augmented with magnesium hydroxide particles, for antiacid properties, which counteract acidifications caused by polymer hydrolysis.

**Advantageous effects of innovation.** The augmented scaffold is characterized by antiacid properties for cartilage tissue engineering during the scaffold degradation.

### BRIEF DESCRIPTION OF THE DRAWINGS

Preferred embodiments of the invention will be described herein below with reference to the drawings. Each figure contains the same numbering for the same or equivalent element.

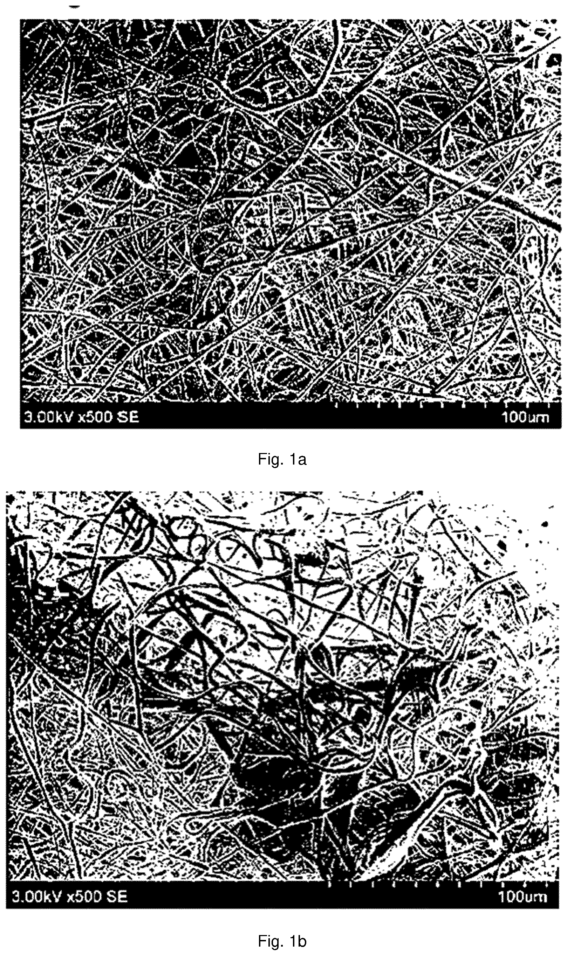

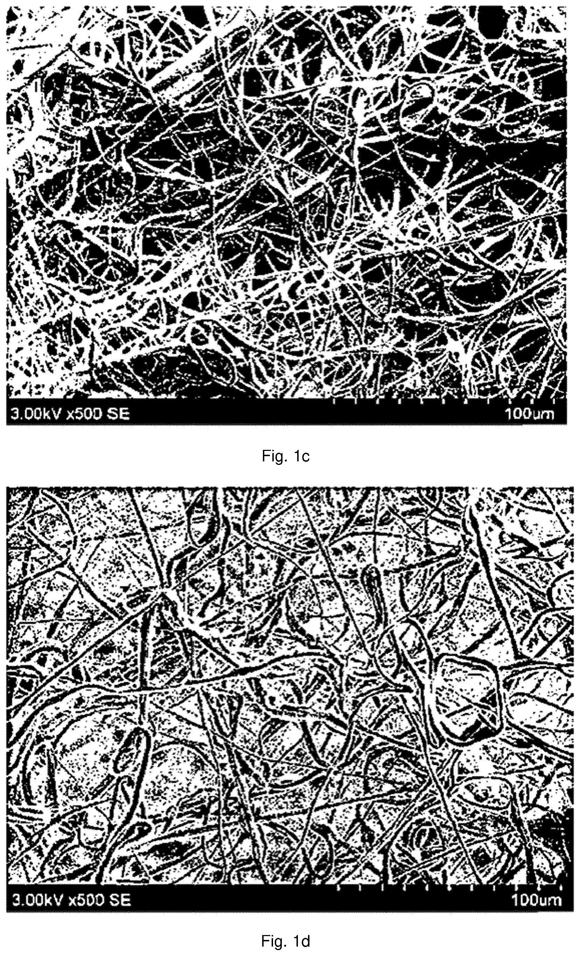

Fig. 1 shows scanned electron microscopy images of examples of scaffolds according to the invention:
   a) PCL:PLGA - 3:1 (wt/wt) - scaffold with added PCL:PLGA, but without added Mg(OH)₂, a control scaffold;
   b) PCL:PLGA - 3:1 (wt/wt) + Mg(OH)₂ 15% - scaffold with added PCL:PLGA and Mg(OH)₂ at 15%;
   c) PCL:PLGA - 3:1 (wt/wt) + Mg(OH)₂ 30% - scaffold with added PCL:PLGA and Mg(OH)₂ at 30%;
   d) PCL:PLGA - 3:1 (wt/wt) + Mg(OH)₂ 45% - scaffold with added PCL:PLGA and Mg(OH)₂ at 45%;
Fig. 2 shows mapping of magnesium (middle) and oxygen (left) elements in: Fig. 2a scaffolds b), Fig. 2b, scaffold c), and in Fig. 2c scaffold d) of Fig.1, according to the invention

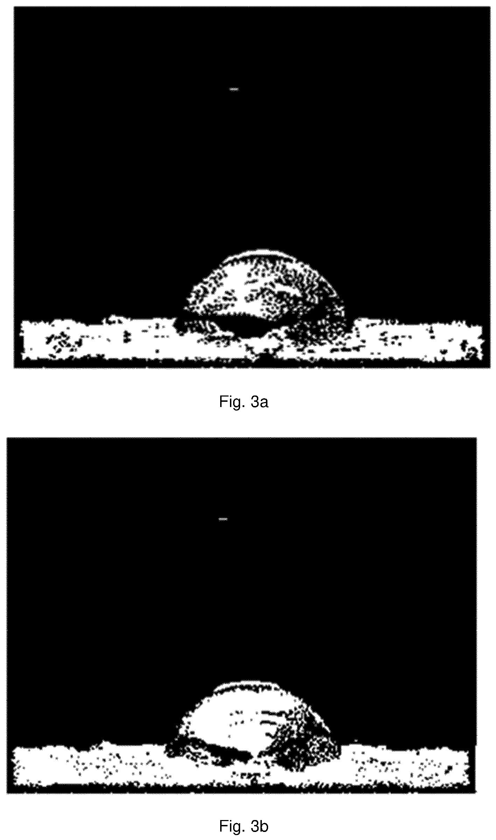

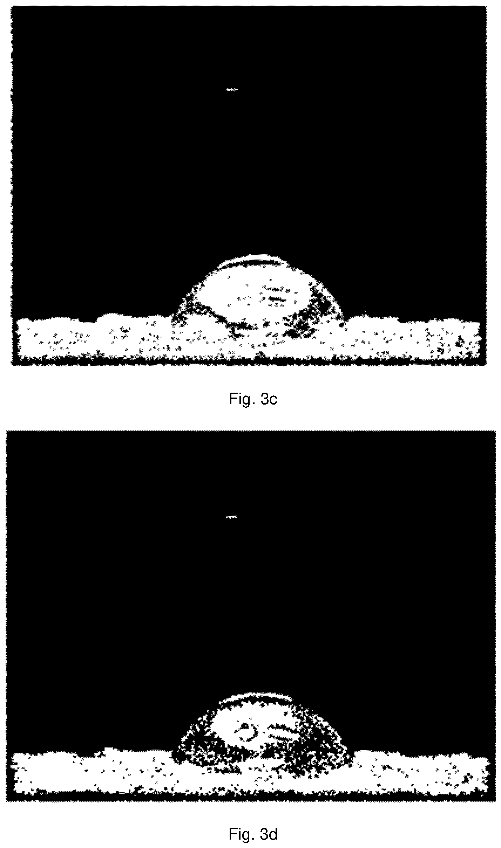

Fig. 3 shows contact angle between the scaffold-water interface in scaffolds: Fig. 3a scaffolds a), Fig. 3b, scaffold b), in Fig. 3c scaffold c), in Fig. 3d scaffold d) of Fig.1 according to the invention.
Fig. 4. shows FTIR spectra in:
   a) scaffold PCL:PLGA - 3:1 (wt/wt);
   b) PCL:PLGA - 3:1 (wt/wt) + Mg(OH)₂ 15%;
   c) PCL:PLGA - 3:1 (wt/wt) + Mg(OH)₂ 30%;
   d) PCL:PLGA - 3:1 (wt/wt) + Mg(OH)₂ 45%.

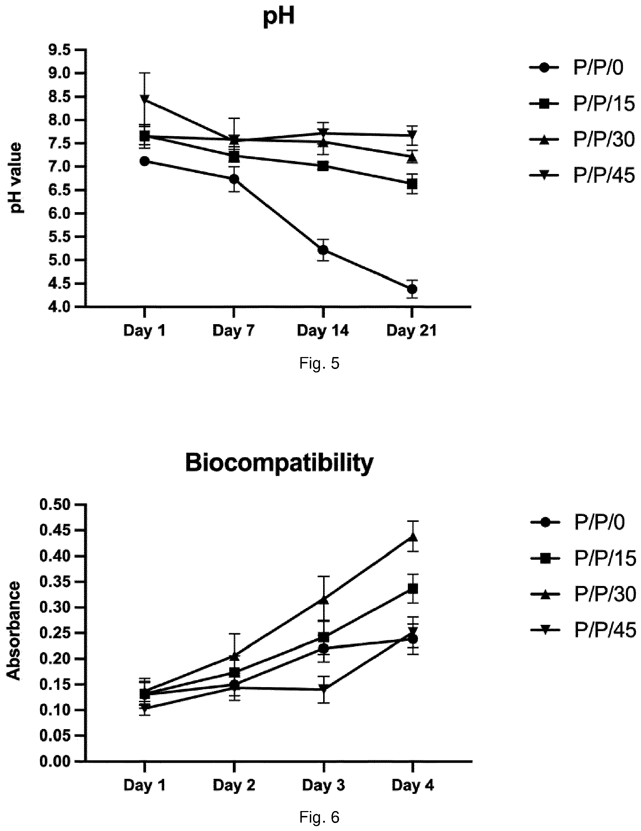

Fig. 5 shows pH change dynamics in the scaffold according to the invention during the scaffold degradation. The untreated P/P/0 scaffold, example shown in Fig.1 a), exhibited the highest change in pH of the media during the 21-day period. Mg(OH)₂ treated P/P/15, example shown in Fig.1b), and P/P/30, example shown in Fig.1c), scaffolds exhibited retained physiological pH values. The P/P/30 exhibited the most consistent pH value dynamics during the 21-day period by retaining it in the physiological area. P/P/45, example shown in Fig.1d), saw the most dramatic initial increase in pH value (acidification), which later subsided however still above the physiological pH value. Values and statistical significances for pH change dynamics are specified in Table 1 and Annex 1 for Table 1.
Fig. 6 shows the biocompatibility level of the experimental scaffolds according to the invention seeded with cells. Viability assay revealed the reduced proliferation of chondrocytes with the highest concentration of Mg(OH)₂ in the scaffold material. P/P/30, example shown in Fig.1c), scaffold provided superior cell proliferation environment and higher chondrocyte proliferation rate in vitro. P/P/45, example shown in Fig.1d) scaffold was comparable to the untreated scaffold, which was marked by the inferior cell proliferation capabilities in the scaffold. Values and statistical significances for biocompatibility levels are specified in Table 2 and Annex 2 for Table 2.

### DETAILED DESCRIPTION OF THE INVENTION

It should be understood that numerous specific details are presented to provide a complete and comprehensible description of the invention embodiment. However, the person skilled in art will understand that the embodiment examples do not limit the application of the invention which can be implemented without these specific instructions. Well-known methods, procedures and components have not been described in detail for the embodiment to avoid misleading. Furthermore, this description should not be considered to be constraining the invention to given embodiment examples but only as one of possible implementations of the invention.

Method of producing a bioactive three-dimensional scaffold according to the invention, for reconstruction of symptomatic, local cartilage defects, comprises use of polycaprolactone (PCL) material for making scaffold by electrospinning method,

Method of producing a bioactive three-dimensional scaffold according to the invention, further comprises selecting and using poly-D-L-lactide-co-glycolide (PLGA) with a lactic acid monomer content of 75% in the polymer and a glycolic acid monomer content of 25%, maintaining a monomer ratio of 75:25. This imparts superior biocompatibility properties to the scaffold because of PCL and the retained mechanical and tensile strength because of the PLGA content.

The method according to the invention further comprises addition of magnesium hydroxide ((Mg(OH)₂) to the scaffold. The magnesium hydroxide is added at 15-45%, in particular 15%, preferably at 45%, and most preferably at 30%.

Method of producing bioactive three-dimensional scaffold preferably comprises supplementing the scaffold with combination of Polycaprolactone, PLGA and Mg(OH)₂ at 30% concentration.

PCL and PLGDA blend improve biocompatibility. Additional Mg(OH)₂ particles incorporation - overcome the in vivo acidification which is the cause of inflammation.

Examples of scaffolds according to the invention prepared by the method according to the invention:
- PCL/PLGA 3/1 (wt/wt)
- PCL/PLGA 3/1 (wt/wt) + Mg(OH)₂ 15% (of PLGA mass)
- PCL/PLGA 3/1 (wt/wt) + Mg(OH)₂ 30% (of PLGA mass)
- PCL/PLGA 3/1 (wt/wt) + Mg(OH)₂ 45% (of PLGA mass)

Preferably polycaprolactone (PCL) of mol wt 80,000 and PLGA- poly(D,L-lactide-co-glycolide) lactide:glycolide (ratio 75:25), mol wt 66,000-107,000 are used.

Preferably bioactive magnesium hydroxide (Mg(OH)₂) substrate are added in the form of nanopowder, <100 nm.

Preferably acetone and dimetilformamide solvents are used in preparation of polymer solution. Preferably the polymer solution concentration is at 20% wt/v. Preferably solvent ratio of acetone:dimetilformamide is 2:3 v/v. For example, to create a solution of 10 ml, 4ml of acetone and 6 ml of dimetilformamide 6 ml (ACE/DMF, 2/3, v/v) are used. Polymer is mixed by combining 1,5 g PCL with 0,5 g PLGA, total at 2 g (PCL/PLGA, 3/1, wt/wt) of polymer.

Electrospining parameters for electrospinning a scaffold according to the invention are preferably as follows: polymer solution supply flow 2.3 ml/h; needle no. 21; distance between needle and collector 15 cm; voltage between needle and collector 22 kV; ambient temperature 30°C; relative humidity 40%. Aluminum foil substrate. The needle moves in the x-axis at 5 cm intervals. The polymer solution is supplied with a Teflon hose. As an example, one sample is formed from 10 ml solution. Dry ice is added to the collector every 25 minutes.

Scaffolds after electrospinning are lyophilized by freezing the electrospun scaffolds in a freezer preferably at -20°C for 24 h. Frozen samples are lyophilized under a vacuum of 2 × 10⁻⁶ mPa at -40°C for 72 h.

Preferably, scaffolds after lyophilization are stored in glass airtight vials in the dark at 20-25°C and 40-60% relative humidity.

In the final step the examples of scaffolds according to the invention were seeded with stem cells. Cell content on one scaffold is restricted to 1×10⁶ cells to 1 cm² area of the scaffold. After trypsinization with Tryple Select, second passage cells are counted and resuspended in vials with culture medium. Cells are seeded in 40 µl doses on presterilized scaffolds in a dropwise fashion. Seeded scaffolds are placed for 2 h in the CO₂ incubator. The seeding of the cells enhances the cartilage restorative properties of the scaffolds, by allowing the cellular adhesion and subsequent proliferation on the scaffolds when in use in therapy. The remaining medium is added to support the effective cell growth and nutrient intake and metabolic waste disposal.

Seeded scaffolds are cultured up to 21 days. Medium is changed every 3-4 days. Scaffolds with cells are harvested after predetermined endpoints for pH, biocompatibility, type II collagen and TNF-a gene expression studies.

At every defined time interval, pH of the media is assessed with a pH meter.

The morphology of the examples of scaffolds according to the invention can be analyzed, for example, by using scanning electron microscopy, e.g. SEM S-3400N, Hitachi Ltd, Japan. Analysis of magnesium and oxygen atoms can be performed, for example, by electron dispersive X-ray spectroscopy (EDS) using the same equipment as SEM at a voltage of 15 kV and a working distance of 10 mm. Characteristics (scaffold filament diameter, filament thickness dispersion, filament length) of manufactured scaffolds are presented in Tables 3-a), 3-b).

Mechanical properties of examples of scaffolds according to the invention can be determined, for example, by using a universal material testing machine, e.g. BDO-FB 0.5 TH, Zwick GmbH & Co, Germany, in a controlled environment according to ASTM D 882 standard. For example, rectangular samples with a length of 60 mm, a width of 14 mm and a thickness of 0.15 mm can be used for the measurements.

The hydrophilicity of examples of scaffolds according to the invention can be measured, for example, by using a Theta Lite TL 101 optical tensiometer, e.g. Biolin Scientific, Finland).

Chemical changes in examples of scaffolds according to the invention can be analyzed, for example, by using the Fourier Transform Infrared (FTIR) spectra, e.g. Perkin-Elmer Frontier, USA, in the range of 4000 to 650 cm⁻¹.

SEM photographs of the developed and manufactured examples of scaffolds according to the invention are shown in Figures 1-a), 1b), 1c), 1d).. From the SEM photographs it is evident that the layout of the scaffolds consists of a dispersed microfiber structure. In all layouts, the strands are arranged in a random order, the strands are not oriented in one direction.

### Elemental map of magnesium and oxygen, of the examples of the scaffolds according to the invention:

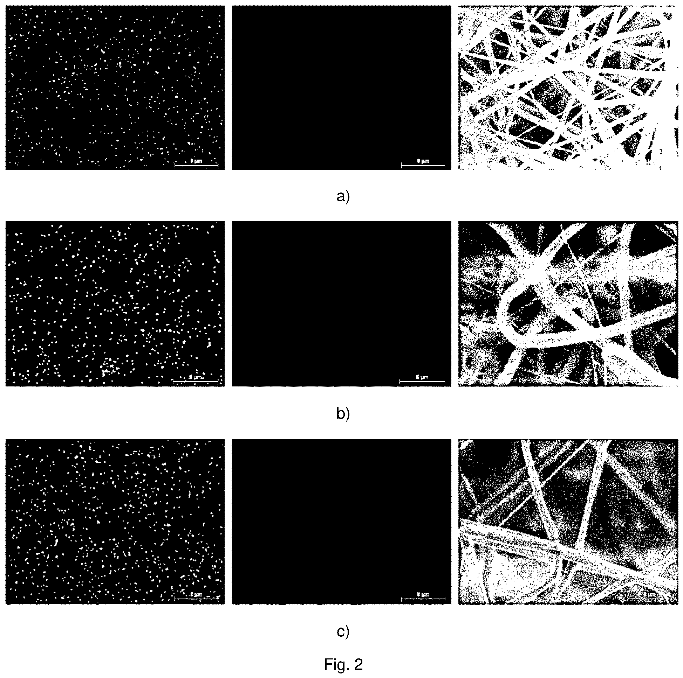

Analysis of magnesium and oxygen elements in the examples of scaffolds PCL/PLGA 3/1 (wt/wt) + Mg(OH)₂ 15% (of PLGA mass), PCL/PLGA 3/1 (wt/wt) + Mg(OH)₂ 30% (of PLGA mass), PCL/PLGA 3/1 (wt/wt) + Mg(OH)₂ 45% (of PLGA mass) are shown in Fig. 2. The right side shows SEM image of a small portion of the layout, with a map of magnesium atoms in the middle and a map of oxygen atoms on the left. It can be seen that both magnesium and oxygen atoms in the scaffolds' fibers are evenly distributed, from which it can be concluded that the magnesium hydroxide particles were properly dispersed in the polymer solution and did not adhere to the agglomerates during the production of the scaffolds.

### Mechanical properties of the examples of the scaffolds according to the invention:

Tensile force measurements of the developed and manufactured examples of the scaffolds according to the invention are shown in Table 4. Scaffold No. 1, which consisted only of a mixture of PCL and PLGA 3/1, has a tensile force of 77 ± 6 Pa. The tensile force of the scaffolds with magnesium hydroxide additive ranged from 201 ± 42 Pa to 383 ± 67 Pa. The tensile force of the scaffolds with nicotinic acid additive ranged from 65 ± 7 Pa to 140 ± 28 Pa. Although addition of magnesium hydroxide particles impairs the tensile properties of the scaffolds, but improves performance of biological function.

### Hydrophilicity of the examples of the scaffolds according to the invention:

The wetting angle results of the scaffold no1. made of a mixture of PCL and PLGA was 64.7 °. The addition of magnesium hydroxide particles in the scaffolds reduced water wetting angle from 61.3 ° (15% magnesium hydroxide particles) to 53.4 ° (45% particles). All examples of scaffolds according to the invention have hydrophilic properties. The wetting angle results of the scaffold no1. made of a mixture of PCL and PLGA was 64.7 °.

### Functional groups of the surface of layout threads:

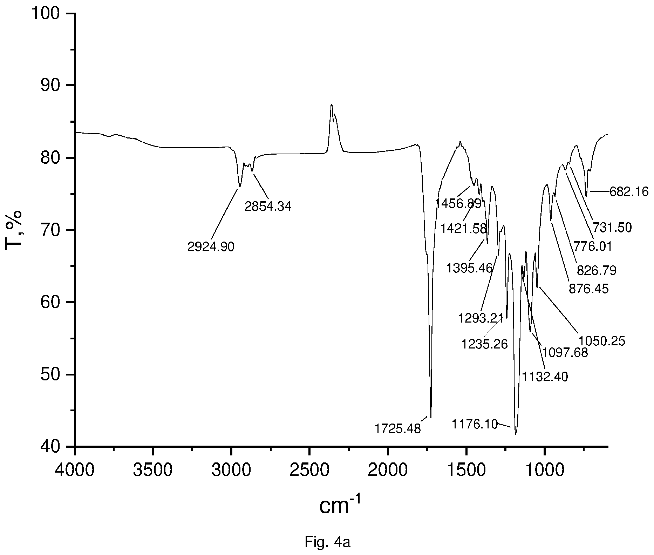

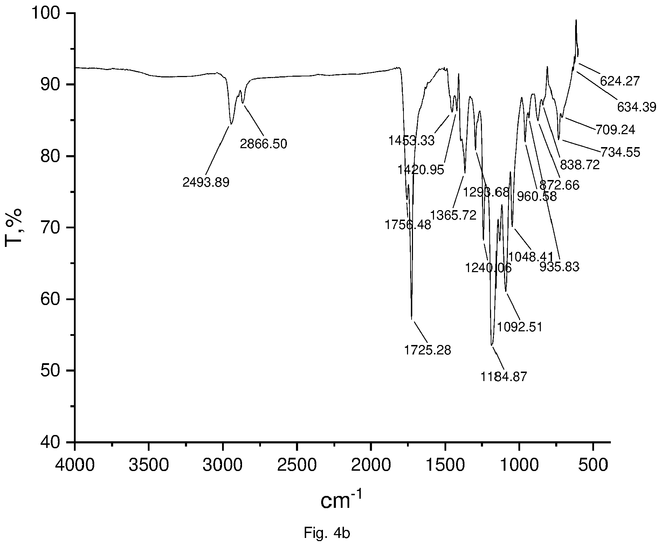

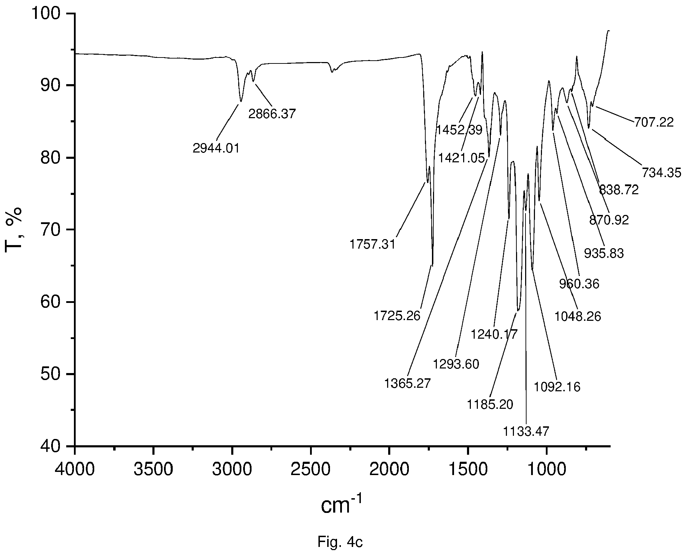

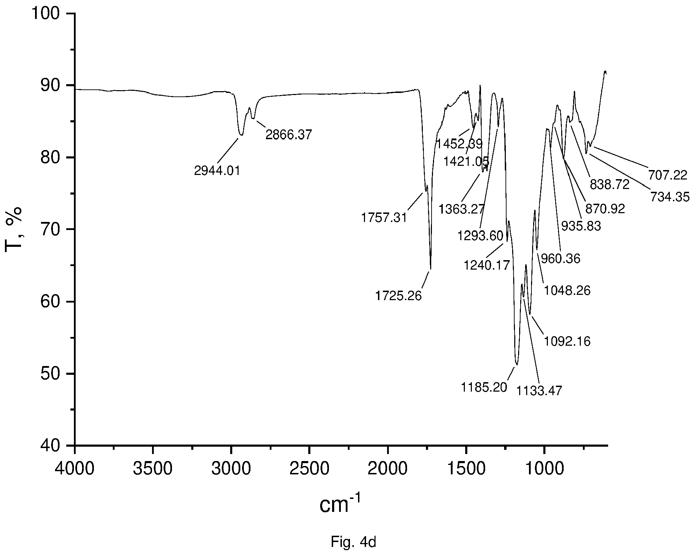

The FTIR spectra of the examples of the scaffolds according to the invention: are presented in Figures 4-a), 4-b), 4-c), 4-d). This analysis allows to determine the presence of functional groups on the surface of the layout threads.

FTIR spectra of all examples of the scaffolds according to the invention have peaks in the range 3000-3700 cm-1, which are characteristic of -OH functional group fluctuations. This peak is broad in nature with low intensity, it is characteristic of the -OH peak in the FTIR spectrum.

### Biocompatibility testing of examples of scaffolds according to the invention:

All experimental procedures were approved and conducted according to the standard guidelines and protocols set by the Kaunas Regional Biomedical Research Ethics Committee. An informed signed content was obtained from the patient. Human cartilage tissue was collected during routine knee reconstruction surgery procedure as a waste material. Briefly, a cartilage biopsy was washed extensively, and digested with collagenase overnight. Isolated cells were plated and cultured in Dulbecco's modified eagle medium supplemented with 10% foetal bovine serum, gentamicin at 37 °C in a humid atmosphere with 5% CO₂. Cells were harvested when reached 80-90% confluence.

Scaffolds were soaked in proliferation medium one day before seeding.

Characteristics of scaffolds according to the invention are as follows:
comprises a cylindrical shape, preferably having diameter of 6 mm and a height of 1 mm);
morphological composition of the scaffolds comprises a microfiber structure in which filaments are arranged in a random direction, i.e. non-oriented;
average filament diameter in the scaffolds is preferably in the range from 1×10⁻⁶ to 1×10⁻⁵ m;
statistical dispersion of scaffolds' filament thickness is preferably 90% variance;
scaffolds' filament thread length is preferably 90th percentile value 6×10⁻⁵ m;
scaffolds' porosity is preferably 85%;
mechanical tensile properties of the scaffolds are preferably 0.6 Pa;
hydrophilicity of the scaffolds is preferably wetting angle 70°;
Surface of the scaffolds comprises hydroxyl groups attached thereto.

The examples of the scaffolds according to the invention are further characterized by extent of degradation of the scaffolds. pH of untreated scaffold P/P/0, example shown in Fig.1a), did not change for the 1^{st} day. Other scaffolds saw an increase in pH values, with respect to the concentration of Mg(OH)₂ used. The initial day 1 saw an acidification of the media, which was later gradually reduced. P/P/45, example shown in Fig.1d), saw the most dramatic increase in pH due to the highest Mg(OH)₂ concentration mixed in the scaffold.

The P/P/30, example shown in Fig.1c), allowed for the most consistent pH change dynamics during the 21-day period retaining it in the physiological area. Therefore, the sustained physiological pH acidity in the peri-scaffold area might have the most compatible environment for the desirable three-dimensional cell proliferation, and the highest neutralizing effect on the scaffold degradation by-products as shown in Table 1 and Fig. 1.

| **Table 1. pH** | | | | | | | | |
|---|---|---|---|---|---|---|---|---|
| | **P/P/0** | | **P/P/15** | | **P/P/30** | | **P/P/45** | |
| | **Average** | **SD** | **Average** | **SD** | **Average** | **SD** | **Average** | **SD** |
| **Day 1** | 7,12 | 0,10 | 7,67 | 0,20 | 7,65 | 0,25 | 8,43 | 0,58 |
| **Day 7** | 6,73 | 0,27 | 7,23 | 0,14 | 7,58 | 0,46 | 7,55 | 0,12 |
| **Day 14** | 5,22 | 0,23 | 7,02 | 0,08 | 7,53 | 0,27 | 7,72 | 0,23 |
| **Day 21** | 4,38 | 0,19 | 6,63 | 0,21 | 7,22 | 0,13 | 7,67 | 0,21 |
| | | | | | | | | |

| **Annex 1 for Table 1** | |
|---|---|
| **Condition** | **Significant difference** |
| Day 1:P/P/0 vs. Day 1 :P/P/15 | 0,0352 |
| Day 1:P/P/0 vs. Day 1 :P/P/30 | 0,0485 |
| Day 1:P/P/0 vs. Day 1 :P/P/45 | <0,0001 |
| Day 1:P/P/0 vs. Day 14:P/P/0 | <0,0001 |
| Day 1:P/P/0 vs. Day 21 :P/P/0 | <0,0001 |
| Day 1:P/P/15 vs. Day 1 :P/P/45 | 0,0002 |
| Day 1:P/P/15 vs. Day 14:P/P/15 | 0,0042 |
| Day 1:P/P/15 vs. Day 21 :P/P/15 | <0,0001 |
| Day 1 :P/P/30 vs. Day 1 :P/P/45 | 0,0002 |
| Day 1 :P/P/45 vs. Day 7:P/P/45 | <0,0001 |
| Day 1 :P/P/45 vs. Day 14:P/P/45 | 0,0008 |
| Day 1 :P/P/45 vs. Day 21 :P/P/15 | <0,0001 |
| Day 1 :P/P/45 vs. Day 21 :P/P/45 | 0,0002 |
| | |
| Day 7:P/P/0 vs. Day 7:P/P/30 | <0,0001 |
| Day 7:P/P/0 vs. Day 7:P/P/45 | <0,0001 |
| Day 7:P/P/0 vs. Day 14:P/P/0 | <0,0001 |
| Day 7:P/P/0 vs. Day 21 :P/P/0 | <0,0001 |
| Day 7:P/P/15 vs. Day 21 :P/P/15 | 0,0127 |
| | |
| Day 14:P/P/0 vs. Day 14:P/P/15 | <0,0001 |
| Day 14:P/P/0 vs. Day 14:P/P/30 | <0,0001 |
| Day 14:P/P/0 vs. Day 14:P/P/45 | <0,0001 |
| Day 14:P/P/0 vs. Day 21 :P/P/0 | <0,0001 |
| Day 14:P/P/15 vs. Day 14:P/P/45 | 0,0013 |
| | |
| Day 21 :P/P/0 vs. Day 21 :P/P/15 | <0,0001 |
| Day 21 :P/P/0 vs. Day 21 :P/P/30 | <0,0001 |
| Day 21 :P/P/0 vs. Day 21 :P/P/45 | <0,0001 |
| Day 21 :P/P/15 vs. Day 21 :P/P/30 | 0,018 |
| Day 21 :P/P/15 vs. Day 21 :P/P/45 | <0,0001 |

### Biocompatibility:

Chondrocytes were used to assess the biocompatibility of the prepared scaffolds. To evaluate the cytocompatibility of the prepared Scaffolds and the viability of the seeded chondrocytes, w Presto Blue viability test was used. 125,000 chondrocytes were suspended in 25 µl of media and seeded on the respective scaffolds in a 6-well plate. Scaffolds with cells were incubated for 60 minutes before the addition of the additional 2 ml of media. The Presto blue assay was performed at a predetermined time (1, 2, 3, and 4 days). The absorbance of the collected Presto blue solution was measured for 5s at 540 nm using a microplate reader with no shaking.

The biocompatibility and cell proliferation on the examples of scaffolds according to the invention were evaluated using human chondrocytes. The *in vitro* Presto blue results demonstrated that the proliferation of chondrocytes is decreased with the higher Mg(OH)₂ concentration.

P/P/30 scaffold provided superior cell proliferation environment by modulating the cytocompatibility within the acidic scaffold. P/P/45 scaffold was comparable to the untreated scaffold, which was marked by the inferior cell proliferation capabilities in both of these carriers.

The scaffold retained and increased the biological scaffold to promote cell survival against the toxicity associated with acidic by-products shown in Table 2 and Fig. 2.

| **Table 2. Biocompatibility** | | | | | | | | |
|---|---|---|---|---|---|---|---|---|
| | **P/P/0** | | **P/P/15** | | **P/P/30** | | **P/P/45** | |
| | **Average** | **SD** | **Average** | **SD** | **Average** | **SD** | **Average** | **SD** |
| **Day 1** | 0,13 | 0,03 | 0,13 | 0,02 | 0,14 | 0,03 | 0,10 | 0,01 |
| **Day 7** | 0,15 | 0,02 | 0,17 | 0,03 | 0,21 | 0,04 | 0,14 | 0,02 |
| **Day 14** | 0,22 | 0,03 | 0,24 | 0,03 | 0,32 | 0,04 | 0,14 | 0,03 |
| **Day 21** | 0,24 | 0,03 | 0,34 | 0,03 | 0,44 | 0,03 | 0,25 | 0,03 |

| **Annex 2 for Table 2** | |
|---|---|
| **Condition** | **Significant difference** |
| Day 1 :P/P/0 vs. Day 14:P/P/0 | <0,0001 |
| Day 1:P/P/0 vs. Day 21 :P/P/0 | <0,0001 |
| Day 1 :P/P/15 vs. Day 14:P/P/15 | <0,0001 |
| Day 1 :P/P/15 vs. Day 21 :P/P/15 | <0,0001 |
| Day 1:P/P/30 vs. Day 7:P/P/30 | 0,0078 |
| Day 1:P/P/30 vs. Day 14:P/P/30 | <0,0001 |
| Day 1:P/P/30 vs. Day 21 :P/P/30 | <0,0001 |
| Day 1:P/P/45 vs. Day 21 :P/P/45 | <0,0001 |
| | |
| Day 7:P/P/0 vs. Day 14:P/P/0 | 0,0078 |
| Day 7:P/P/0 vs. Day 21 :P/P/0 | 0,0001 |
| Day 7:P/P/15 vs. Day 14:P/P/15 | 0,0107 |
| Day 7:P/P/15 vs. Day 21 :P/P/15 | <0,0001 |
| Day 7:P/P/15 vs. Day 21 :P/P/30 | <0,0001 |
| Day 7:P/P/30 vs. Day 7:P/P/45 | 0,0271 |
| Day 7:P/P/30 vs. Day 14:P/P/30 | <0,0001 |
| Day 7:P/P/30 vs. Day 21 :P/P/30 | <0,0001 |
| Day 7:P/P/45 vs. Day 21 :P/P/45 | <0,0001 |
| | |
| Day 14:P/P/0 vs. Day 14:P/P/30 | <0,0001 |
| Day 14:P/P/0 vs. Day 14:P/P/45 | 0,001 |
| Day 14:P/P/15 vs. Day 14:P/P/30 | 0,0028 |
| Day 14:P/P/15 vs. Day 14:P/P/45 | <0,0001 |
| Day 14:P/P/15 vs. Day 21 :P/P/15 | <0,0001 |
| Day 14:P/P/30 vs. Day 14:P/P/45 | <0,0001 |
| Day 14:P/P/30 vs. Day 21 :P/P/30 | <0,0001 |
| Day 14:P/P/45 vs. Day 21 :P/P/45 | <0,0001 |
| | |
| Day 21 :P/P/0 vs. Day 21 :P/P/15 | <0,0001 |
| Day 21 :P/P/0 vs. Day 21 :P/P/30 | <0,0001 |
| Day 21 :P/P/15 vs. Day 21 :P/P/30 | <0,0001 |
| Day 21 :P/P/15 vs. Day 21 :P/P/45 | 0,0003 |
| Day 21 :P/P/30 vs. Day 21 :P/P/45 | <0,0001 |

**Table 3.**

| |
|---|
| **Table 3a. Scaffold filament diameter, filament thickness dispersion, filament length** |
| 1. PCL/PLGA 3/1 (wt/wt) - 5,4±1,2 × 10⁻⁶ m, 92%, 6,5×10⁻⁵ m. |
| 2. PCL/PLGA 3/1 (wt/wt) + Mg(OH)₂ 15% - 5,0±1,3 × 10⁻⁶ m, 93%, 6,3×10⁻⁵ m. |
| 3. PCL/PLGA 3/1 (wt/wt) + Mg(OH)₂ 30% - 4,6±0,9 × 10⁻⁶ m, 91%, 6,4×10⁻⁵ m. |
| 4. PCL/PLGA 3/1 (wt/wt) + Mg(OH)₂ 45% - 5,2±2,5 × 10⁻⁶ m, 94%, 6,2×10⁻⁵ m. |
| **Table 3b. Scaffold porosity** |
| 1. PCL/PLGA 3/1 (wt/wt) - 87,1% |
| 2. PCL/PLGA 3/1 (wt/wt) + Mg(OH)₂ 15% -86,5% |
| 3. PCL/PLGA 3/1 (wt/wt) + Mg(OH)₂ 30% -88,2% |
| 4. PCL/PLGA 3/1 (wt/wt) + Mg(OH)₂ 45% - 87,5% |

**Table 4.**

| Scaffolds | Tension, Pa | |
|---|---|---|
| | Average | StDev |
| 1. PCL/PLGA 3/1 (wt/wt) | 77 | 6 |
| 2. PCL/PLGA 3/1 (wt/wt) + Mg(OH)₂ 15% | 383 | 67 |
| 3. PCL/PLGA 3/1 (wt/wt) + Mg(OH)₂ 30% | 175 | 35 |
| 4. PCL/PLGA 3/1 (wt/wt) + Mg(OH)₂ 45% | 210 | 42 |

### NON-PATENT LITERATURE

1. Anders S, Volz M, Frick H, et al. A Randomized, controlled trial comparing autologous matrix-induced chondrogenesis (AMIC®) to microfracture: analysis of 1- and 2-year follow-up data of 2 centers. Open Orthop J 2013; 7: 133-143.
2. Gudas R, Kalesinskas RJ, Kimtys V, et al. A prospective randomized clinical study of mosaic osteochondral autologous transplant versus microfracture for the treatment of osteochondral defects in the knee joint in young athletes. Arthroscopy 2005; 21: 1066-1075.
3. Kim YS, Choi YJ, and Koh YG. Mesenchymal stem cell implantation in knee osteoarthritis: an assessment of the factors influencing clinical outcomes. Am J Sports Med 2015; 43(9): 2293-2301.
4. Knutsen G, Engebretsen L, Ludvigsen TC, et al. Autologous chondrocyte implantation compared with microfracture in the knee: a randomized trial. J Bone Joint Surg Am 2004; 86: 455-464.
5. Behery OA, Harris JD, Karnes JM, et al. Factors influencing the outcome of autologous chondrocyte implantation: a systematic review. Knee Surg 2013; 26(3): 203-211.
6. L. Averous, L. Moro, P. Dole, C. Fringant, Properties of thermoplastic blends: starch-polycaprolactone, Polymer (Guildf). 41 (2000) 4157-4167.
7. H.K. Makadia, S.J. Siegel, Poly Lactic-co-Glycolic Acid (PLGA) as Biodegradable Controlled Drug Delivery Carrier, Polymers (Basel). 3 (2011) 1377.
8. H. Song, Y. Kim, J. Park, M. Park, S. Lyu, Y. Koh, J. Heo, D. Lee, K. Park, Biocompatible nanoparticle PLGA is a noble safe delivery system for embryo development and next generations, Fertil. Steril. 108 (2017) e154.
9. K. Park, S. Skidmore, J. Hadar, J. Garner, H. Park, A. Otte, B.K. Soh, G. Yoon, D. Yu, Y. Yun, B.K. Lee, X. Jiang, Y. Wang, Injectable, long-acting PLGA formulations: Analyzing PLGA and understanding microparticle formation, J. Control. Release. 304 (2019) 125-134.
10. Houchin M.; Topp E. Chemical degradation of peptides and proteins in PLGA: a review of reactions and mechanisms. J. Pharm. Sci. 2008, 97 (7), 2395.
11. A. Abdal-hay, N.T. Raveendran, B. Fournier, S. Ivanovski, Fabrication of biocompatible and bioabsorbable polycaprolactone/ magnesium hydroxide 3D printed scaffolds: Degradation and in vitro osteoblasts interactions, Compos. Part B Eng. 197 (2020) 108158.
12. T. M. Bedair, Yun Heo, Jungju Ryu, H. M. Bedair, Wooram Park, D. Keun Han, Biocompatible and functional inorganic magnesium ceramic particles for biomedical applications, Biomater. Sci. 9 (2021) 1903-1923.
13. Pestka JM, Feucht MJ, Porichis S, et al. Return to sports activity and work after autologous chondrocyte implantation of the knee: which factors influence outcomes? Am J Sports Med 2016; 44(2): 370-377.
14. Solheim E, Oyen J, Hegna J, et al. Microfracture treatment of single or multiple articular cartilage defects of the knee: a 5-year median follow-up of 110 patients. Knee Surg Sports Traumatol Arthrosc 2010; 18(4): 504-508.
15. S.M. Espinoza, H.I. Patil, E.S.M. Martinez, R.C. Pimentel, P.P. Ige, Poly-ε-caprolactone (PCL), a promising polymer for pharmaceutical and biomedical applications: Focus on nanomedicine in cancer, Https://Doi.Org/10.1080/00914037.2018.1539990. 69 (2019) 85-126
16. R.M. Mohamed, K. Yusoh, A Review on the Recent Research of Polycaprolactone (PCL), Adv. Mater. Res. 1134 (2016) 249-255..
17. Biomaterials and Nanotechnology for Tissue Engineering - Google Books, (n.d.). https://books.google.lt/books?hl=en&lr=&id=MSgNDgAAQBAJ&oi=fnd&pg=PP1&ots=0OYL-PNiZe&sig=MhJg4Q61ryPOkh2PuvjA5SWU6IY&redir_esc=y#v=onepage&q&f=false
18. H. J, G. N, J. H, K. M, R. A, M. E, S. N, Three-Dimensional Printed PCL-Based Implantable Prototypes of Medical Devices for Controlled Drug Delivery, J. Pharm. Sci. 105 (2016) 2665-2676.S. Jin, X. Xia, J. Huang, C. Yuan, Y. Zuo, Y. Li, J. Li, Recent advances in PLGA-based biomaterials for bone tissue regeneration, Acta Biomater. 127 (2021) 56-79.
20. M. Pallavi, J. Waterman, Y. Koo, J. Sankar, Y. Yun, In Vitro Cytotoxicity of Possible Corrosion Products from Mg-Based Biodegradable Metals: Magnesium Oxide and Magnesium Hydroxide Nanoparticles, Appl. Sci. 2019, Vol. 9, Page 4304. 9 (2019) 4304.

## Claims

1. Method for producing an electrospinned three-dimensional scaffold for cartilage regeneration, comprising electrospinning a scaffold **characterised by**
- use of polycaprolactone (PCL) material for making scaffold by electrospinning;
- selecting and using poly-D-L-lactide-co-glycolide (PLGA) with a lactic acid monomer content;
- addition of Mg(OH)₂ to the scaffold;
wherein acetone and simetilformamide solvents are used in preparation of polymer solution;
- lyophilizing by freezing the electrospun scaffold in a freezer preferably at -20°C for 24 h;
- lyophilizing frozen samples under a vacuum of 2 × 10⁻⁶ mPa at -40°C for 72 h;
- storing scaffold after lyophilization in glass airtight vials in the dark at 20-25°C and 40-60% relative humidity.

2. Method according to claim 1, where poly-D-L-lactide-co-glycolide (PLGA) with a lactic acid monomer content is of 75% in the polymer and a glycolic acid monomer content of 25%, maintaining a monomer ratio of 75:25.

3. Method according to claim 1 or 2 wherein the Mg(OH)₂ is added at 15-45%, in particular 15%, preferably at 45%, and most preferably at 30%.

4. Method according to any one claims of 1 - 3, wherein polycaprolactone (PCL) of mol wt 80,000 and PLGA- poly(D,L-lactide-co-glycolide) lactide:glycolide (ratio 75:25), mol wt 66,000-107,000 are used.

5. Method according to any one claims of 1 - 4, wherein the bioactive Mg(OH)₂ substrate is added in the form of nanopowder, <100 nm.

6. Method according to any one claims of 1 - 5, wherein acetone and dimetilformamide solvents are used in preparation of polymer solution having concentration at 20% wt/v..

7. Method according to any one claims of 1 - 6, wherein solvent ratio of acetone:dimetilformamide is 2:3 v/v.

8. Method according to any one claims of 1 - 7, wherein electrospining parameters for electrospinning a scaffold according to the invention are as follows:
- polymer solution supply flow 2.3 ml/h;
- needle no. 21;
- distance between needle and collector 15 cm;
- voltage between needle and collector 22 kV;
- ambient temperature 30°C;
- relative humidity 40%;
- using Aluminum foil substrate;
- the needle moves in the x-axis at 5 cm intervals;
- the polymer solution is supplied with a Teflon hose;
- adding drying ice to the collector every 25 minutes.

9. A three-dimensional scaffold for cartilage regeneration, produced by method according to any one of claims 1-9, comprising electrospun filaments, **characterized in that** it comprises polycaprolactone, poly-D-L-lactide-co-glycolide, Mg(OH)₂ and stem cells.

10. A three-dimensional scaffold according to claim 9, where the scaffold comprises PCL/PLGA 3/1 (wt/wt) + Mg(OH)₂ 15% (of PLGA mass).

11. A three-dimensional scaffold according to claim 9, where the scaffold comprises PCL/PLGA 3/1 (wt/wt) + Mg(OH)₂ 30% (of PLGA mass).

12. A three-dimensional scaffold according to claim 9, where the scaffold comprises PCL/PLGA 3/1 (wt/wt) + Mg(OH)₂ 45% (of PLGA mass).
